Europäisches Patentamt

European Patent Office

Office européen des brevets

(11) Publication number: **0 210 684**
**A1**

# (12) EUROPEAN PATENT APPLICATION

(21) Application number: **86201154.1**

(22) Date of filing: **01.07.86**

(51) Int. Cl.4: **A61K 49/02** , C07B 59/00 , C07K 15/00

(30) Priority: **31.07.85 NL 8502161**

(43) Date of publication of application:
**04.02.87 Bulletin 87/06**

(84) Designated Contracting States:
**AT BE CH DE FR GB IT LI NL SE**

(71) Applicant: **MALLINCKRODT, INC.(a Missouri corporation)**
**675 McDonnell Boulevard P.O. Box 5840**
**St. Louis Missouri 63134(US)**

(72) Inventor: **Goedemans, Wilhelmus Theodorus, Dr.**
**c/o OCTROOIBUREAU ZOAN B.V. P.O. Box 140**
**NL-1380 AC Weesp(NL)**

(74) Representative: **Swaters, Pieter D. et al**
**OCTROOIBUREAU ZOAN B.V. P.O. Box 140**
**NL-1380 AC Weesp(NL)**

(54) Composition comprising a radioactive metal compound for radioassaying.

(57) The invention relates to a composition suitable for radioassaying, in particular for localising inflammatory processes in the body, which composition comprises a chelate of radioactive indium with lactoferrin as the radioactive compound.

EP 0 210 684 A1

## Composition comprising a radioactive metal compound for radioassaying.

The invention relates to a composition suitable for radioassaying, in particular for localising inflammatory processes in the body, which composition comprises a radioactive metal compound in addition to a pharmaceutically acceptable formulation liquid and optionally an inactive carrier and/or one or more auxiliary substances. The invention further relates to the preparation and to the use of said composition, as well as to a radioactive metal compound to be used for this composition.

It is highly desirable to determine the location of inflammatory processes in warm-blooded living beings without it being necessary to invade the body at the area of the inflammation.

In order to meet this desire, it is suggested in United States Patent 4,360,509 to introduce a chelate of radioactive indium with 8-hydroxyquinoline, for example indium-111 oxinate, in the blood stream of the being. After a given period of time the radioactivity accumulates not only in certain organs such as the liver and the spleen, but also in comparatively large quantities in any inflamed region present. The body may then be subjected to a radioassay in which a suitable imaging technique is used. In this manner, the accumulated radioactivity can simply be detected at the location of an abscess or another inflammation, provided said inflammation is present in a part of the body other than the part in which the radioactivity would usually accumulate to approximately the same extent even in the absence of an inflammation. The use of indium-111 transferrin for imaging tumors is described in an article by Goodwin et al. in J. Nucl. Medic., Vol 24, p. 32 (1983).

It has been found, however, that the specificity with regard to the "target organ", in this case the location of the inflammation, both of indium-111 oxinate and of indium-111 transferrin leaves to be desired. This means that when these known radioactive indium compounds are used, the radioactivity also accumulates in other places in the body than in the place of the inflammation to an extent which impedes the localisation of the inflammatory process, in particular in an early-stage of the development thereof. Now it is very important to be able to detect an inflammatory process in an early stage of its development, because curative measures are most efficacious exactly in that stage.

In an article by Som et al in Eur. J. Nucl. Med. (1983) 8, 491-494 the organ distribution of ruthenium-103 transferrin is compared with that of gallium-67 citrate. From this it appears that the clearance of labelled ruthenium transferrin is considerably slower than that of labelled gallium citrate. A rapid clearance is important because that leads faster to a lower background radiation and hence to a better ratio in radiactivity between "target organ" and background. As is generally known the organ distribution must be ascribed to the chelating agent used, i.c. transferrin with respect to citrate, and not to the radioactive labelled metal atom.

According to Vallabhajosula et al (Eur. J. Nucl. Med. (1983) 8, 354-357), these detrimental properties of transferrin with respect to citrate are also expressed in the tumor uptake. When gallium-67 transferrin is used, 30% less radioactivity was found in the tunor than when gallium-67 citrate was used. According to these authors, gallium-67 lactoferrin proves to be even less suitable: a reduction of the uptake in the tumor of 57% compared with gallium-67 citrate.

Organ distribution studies with a comparison between gallium-67 lactoferrin and gallium-67 transferrin are also described in an article by Anghileri et al in Nucl.-Med. Band XXI, Heft 1, 1982, 8-11. The differences between transferrin and lactoferrin are small: for tumor examination gallium-67 transferrin proves to be slightly more suitable than the corresponding lactoferrin chelate. The uptake in the inflammatory process for both chelates is unfavourable in relation to that in various "organs" such as liver, kidney and bone.

It has furthermore been found that, when indium-111 oxinate and citrate are used, the spleen is heavily burdened because comparatively much radioactivity is deposited in the spleen. For these radioactive compositions the spleen is the critical organ so that an as low as possible uptake of radioactivity in the spleen must be pursued. The same unfavourable spleen burden is found when radiolabelled white bloodcells are used, a method which is frequently used in practice for localising inflammatory processes in the body.

It is the object of the invention to provide a composition suitable for radioassaying which is meant in particular for localising inflammatory processes in the body and which must inter alia satisfy the following conditions: a sufficiently rapid clearance and a low spleen burden.

It was now found surprisingly that this object can be achieved by means of a composition mentioned in the opening paragraph which, according to the present invention comprises a chelate of radioactive indium with lactoferrin as a radioactive metal compound. Such a chelate has been found to be exceptionally selective with respect to abscesses and other inflammations and hence to be excellently suitable for use in a composition for localising inflammatory processes. Furthermore, when this composition is used the spleen burden is comparatively low and the clearance is rapid, as will become apparent from the examples.

The chelate of indium-111 and lactoferrin has proven to be particularly suitable, because the radioisotope used therein has a favourable half-life and suitable radiation characteristics. Moreover indium-111 can be produced in a cyclotron in a simple manner and is amply available. A composition is preferred which comprises the chelate mentioned hereinbefore in a quantity of from approximately 0.005 to 20 mg per ml. Water is preferably used as a carrier material for the composition. For example, the radioactive compound may be incorporated in a physiological saline solution which comprises a buffer, for example, a phosphate buffer or a TRIS-buffer. If desired, the composition may comprise other salts, for example, bicarbonate.

The compositions according to the invention are particularly suitable for in vivo-application, i.e. the compositions are administered directly to the body, namely by introducing them into the blood stream of the living being, in particular a human being. However, the invention is not restricted hereto. The compositions may also be used for the radioactive labelling of blood outside the body, hence semi in vivo, after which the labelled blood is again introduced into the body of the living being. However, the in vivo-application requires fewer operations with radioactive material, is hence faster and simpler and is therefore to be preferred. After a given period after the administration of the composition the radioactivity has accumulated, for example in comparatively large quantities in the inflamed area in question. The body may then be subjected to a radioassay, using a technique of external imaging, to detect the accumulated radioactivity at the location of the inflamed area, provided said area is present in a part of the body other than that in which the radioactivity would usually accumulate to substantially the same extent even in the absence of an inflamed area. The invention therefore also relates to a method of subjecting a warm-blooded living being, in particular a human being, to radioassaying, in particular to localise inflammatory processes in the body. The composition according to the invention is then administered intravenously. The quantity of the administered imaging active substance may be very small, but, of course, must be sufficient to enable detection by external imaging. A quantity of approximately 0.1 to 10 mCi of radioactive material, for example 0.5 to 3 mCi, per 70 kg of body weight has proved suitable for this purpose.

After the radioactive imaging active substance has been introduced into the living being's body, a radioassay can be carried out by using a suitable imaging technique. A detector suitable for gamma rays, for example, a gamma camera, is used. In general, the accumulation of the radioactivity in, for example, the inflamed area, occurs sufficiently after a few hours, to permit the scanning of, for example, the inflamed area already after a few hours counted from the moment the radioactive material has been injected. The life of the imaging active substance, in particular of an indium-111-labelled chelate, however, is sufficiently extended to permit rescanning several days after the injection. On the other hand, when said chelates are applied, this life is not so extended that the substance poses an excessive radiation burden to the body. As already stated hereinbefore, the accumulated radioactivity can be detected, for example, to localise an inflammatory process in the body.

Chelates of radioactive indium with lactoferrin which may be used in a composition according to the invention are new. The invention therefore also relates to a chelate of radioactive indium with lactoferrin, in particular to the indium-111 lactoferrin which is very suitable for use in a composition for radioassaying.

The new chelates may be prepared in a manner which is known per se for the preparation of related compounds. For example, the chelate of radioactive indium with lactoferrin can be prepared by causing a chelate of radioactive indium with a suitable chelating agent, for example, tropolone, pyrithione or 8-hydroxy quinoline, to react with lactoferrin. The reaction is preferably carried out in an aqueous medium at a pH suitable for the reaction and at a reaction or incubation temperature which on the one hand causes the reaction to run off sufficiently rapidly but on the other hand has no detrimental influence on lactoferrin or compounds thereof. The pH may be adjusted by means of a suitable buffer, for example, a phosphate buffer. As an aqueous medium is preferably chosen an, optionally physiological, sodium bicarbonate solution. Bicarbonate has been found to have a particularly favourable influence on the course of the reaction. The incubation is preferably carried out at room temperature or a slightly elevated or reduced temperature.

The invention will now be described in greater detail with reference to the following specific examples.

## EXAMPLE I

Lactoferrin in a quantity of 1.07 mg was dissolved in 1 ml of phosphate-buffered saline. 0.45 ml of an indium-111 tropolonate solution in a diluted sodium bicarbonate solution having a radioactivity of 0.486 mCi was added to said first solution. This mixture was incubated at room temperature for 20 minutes. The coupling of indium-111 to lactoferrin was determined by means of gel chromatography of the coupling product on a Sephadex (registered trademark) G50 column. From this it appeared that the radioactivity had bound quantitatively to the lactoferrin. The formed indium-111 lactoferrin had a specific activity of 0.455 mCi per mg.

The resulting composition containing indium-111-lactoferrin was used for abscess location in the same manner as described in the above-mentioned Netherlands Patent Application 8006869. For that purpose, the composition was administered intravenously into a goat in which bacterial abscesses had been provoked. The next day a scintigraphic image of the goat was made by means of a gamma camera, the abscesses being visible as clear spots.

## EXAMPLE II

Lactoferrin in a quantity of 0.98 mg was dissolved in 1 ml of diluted sodium bicarbonate solution. 0.45 of an indium-111 oxinate solution in a diluted acetate-TRIS buffer solution having a radioactivity of 0.45 mCi were added to said first solution. This mixture was incubated as in Example I, after which the coupling of indium-111 to lactoferrin was determined as in Example I. The radioactivity had bound quantitatively to the lactoferrin. The indium-111 lactoferrin formed had a specific activity of 0.46 mCi per mg.

The resulting composition containing indium-111 lactoferrin could be used for abcess localisation in the same manner as described in Example I.

## EXAMPLE III

The indium-111 lactoferrin-containing composition obtained according to Example I was used for abcess localisation experiments in rats in which abcesses had been provoked artificially. For comparison a corresponding composition of an indium-111-labelled leucocytes suspension was used.

The experiments were carried out on two groups of twelve rats; a bacterial abcess had been provoked in the abdominal cavity of the rats. A composition containing indium-111 lactoferrin was administered intravenously to the rats of one group; the rats of the other group were injected with a composition containing an indium-111-labelled, mixed leucocytes suspension. After 48 hours, the rats of both groups were subjected to a radioassay. From the experiments it appeared that the radioactivity had accumulated in the abcesses of the rats to an extent which for the group of test animals treated with indium-111-lactoferrin on an average was equally large as for the control group, i.e. the group of rats treated with an indium-111-labelled mixed leucocytes suspension. The group of test animals treated with indium-111 lactoferrin, however, distinguished favourably from the control group as regards the clearance and the spleen burden. The ratio of the accumulation of radioactivity in the abcess to that in the blood after 48 hours was on an average 4 for the test animals treated with indium-111 lactoferrin, that of the control group was only 0.2. The accumulation of radioactivity in the spleen in the group of test animals treated with indium-111 lactoferrin was approximately four times as small as that of the control group.

## EXAMPLE IV

In the same manner as described in Example III, an indium-111 oxinate-containing composition was compared with a corresponding composition which contained indium-111 lactoferrin and had been obtained according to Example I. It was found that, in contrast with indium-111 lactoferrin, indium-111 oxinate had not accumulated at all in the abcess of the test animals, but on the contrary had been taken up to a high extent in the spleen.

## Claims

1. A composition suitable for radioassaying, in particular for localising inflammatory processes in the body, which composition comprises a radioactive metal compound in addition to a pharmaceutically acceptable formulation liquid and optionally an inactive carrier and/or one or more auxiliary substances, characterized in that the radioactive metal compound is a chelate of radioactive indium with lactoferrin.

2. A composition as claimed in Claim 1, characterized in that the radioactive metal compound is indium-111 lactoferrin.

3. A composition as claimed in Claim 2, characterized in that the composition comprises the radioactive metal compound in a quantity from approximately 0.005 to 20 mg per ml.

4. A method of preparing a composition as claimed in any of the preceding Claims, characterized in that a chelate of radioactive indium with lactoferrin is brought into a form suitable for radioassaying.

5. A method of subjecting a warm-blooded living being to a radioassay, in particular to localise inflammatory processes in the body, characterized in that a composition as claimed in any of the Claims 1-3 is administered to the being, the quantity of administered radioactivity being sufficient for detection by means of external imaging, and that the being is then subjected to external imaging to detect accumulated radioactivity and to thus determine the location thereof in the body of the being.

6. A method as claimed in Claim 5, characterized in that the radioactive material is administered to the living being in a quantity of approximately 0.1 to 10 millicurie, preferably 0.5 to 3 millicurie, per 70 kg of body weight.

7. A radioactive metal compound to be used for a composition as claimed in any of the Claims 1-3, characterized in that the metal compound is a chelate of radioactive indium with lactoferrin.

8. A chelate of radioactive indium with lactoferrin.

9. Indium-111 lactoferrin.

10. A method of preparing a radioactive metal compound, characterized in that a chelate of radioactive indium with lactoferrin is prepared by causing a chelate of radioactive indium with a suitable chelating agent to react with lactoferrin, preferably in the presence of bicarbonate.

European Patent Office

**PARTIAL EUROPEAN SEARCH REPORT**
which under Rule 45 of the European Patent Convention
shall be considered, for the purposes of subsequent
proceedings, as the European search report

Application number

EP 86 20 1154

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 85, no. 7, August 16, 1976, page 267, ref.no. 43302x; Columbus, Ohio, US J.P. ESQUERRE et al.: "Determining blood volume by the dilution of indium 113m-labeled siderophillin. Preliminary results." & REV. MED. TOULOUSE, 1976, 12(2), 185-9.<br><br>* Abstract * | 1-4,7-10 | A 61 K 49/02<br>C 07 B 59/00<br>C 07 K 15/00 |
| | --- | | |
| A | INT. JOURNAL OF APPLIED RADIATION AND ISOTOPES, vol. 31, no. 12, 1980, pages 747-751, Pergamon Press, Ltd., GB B. BASSE-CATHALINAT et al.: "Direct in vivo study of flowing blood-artificial surface interactions. An original application of dynamic isotopic techniques."<br><br>* Page 747, abstract; page 748, table 1, left-hand column, paragraph 1 * | 1-4,7-10 | TECHNICAL FIELDS SEARCHED (Int. Cl.4)<br><br>A 61 K |

--- 

**INCOMPLETE SEARCH** --- ./.

The Search Division considers that the present European patent application does not comply with the provisions of the European Patent Convention to such an extent that it is not possible to carry out a meaningful search into the state of the art on the basis of some of the claims.

Claims searched completely: 1-4,7-10
Claims searched incompletely:
Claims not searched: 5,6
Reason for the limitation of the search:

Method for treatment of the human or animal body by surgery or therapy and diagnostic method practised on the human or animal body (see art. 52(4) of the European Patent Convention).

| Place of search | Date of completion of the search | Examiner |
|---|---|---|
| The Hague | 06-11-1986 | CHARLES |

EPO Form 1505.1 03.82

| | DOCUMENTS CONSIDERED TO BE RELEVANT | | CLASSIFICATION OF THE APPLICATION (Int Cl 4) |
|---|---|---|---|
| Category | Citation of document with indication, where appropriate, of relevant passages | Relevant to claim | |
| A | NL-A-80 06 869 (BYK-MALLINCKRODT CIL B.V.)<br><br>* Page 1, lines 1-4; page 3, lines 8-29, page 4, line 12 - page 5, line 1; claims 1-5 *<br><br>& US-A-4 360 509 (Cat. D)<br><br>--- | 1-4,7-10 | |
| A | JOURNAL OF NUCLEAR MEDICINE, vol. 22, 1981, pages 32-37 R. WEINER et al.: "Lactoferrin: Its role as a Ga-67-binding protein in polymorphonuclear leukocytes."<br><br>* Page 32, abstract; page 36, left-hand column, line 45 - right-hand column, line 22 *<br><br>--- | 1-4 | TECHNICAL FIELDS SEARCHED (Int Cl 4) |
| D,A | CHEMICAL ABSTRACTS, vol. 96, no. 25, June 21, 1982; page 332, ref.no. 213343h; Columbus, Ohio, US L.J. ANGHILERI et al.: "In vivo distribution of carrier protein/ gallium-67-complexes." & NUKLEARMEDIZIN (Stuttgart), 1982, 21(1), 8-11.<br><br>* Abstract *<br><br>--- | 1-4,7-10 | |
| A | EP-A-0 083 129 (BYK-MALLINCKRODT CIL B.V.)<br><br>------- | | |